# EUROPÄISCHE PATENTSCHRIFT

(11) **EP 1 017 337 B1**
(45) Veröffentlichungstag und Bekanntmachung des Hinweises auf die Patenterteilung: **07.05.2003**
(21) Anmeldenummer: 98902948.3
(22) Anmeldetag: 10.01.1998
(51) Int. Cl.: A61F 2/18

(54) **MITTELOHRPROTHESE**
MIDDLE EAR PROSTHESIS
PROTHESE DE L'OREILLE MOYENNE

(30) Priorität: 13.01.1997 DE 19700813
(43) Veröffentlichungstag der Anmeldung: 12.07.2000
(73) Patentinhaber: Stennert, Eberhard, 50935 Köln (DE)
(72) Erfinder: STENNERT, Eberhard, D-50935 Köln (DE); WALGER, Martin, D-50354 Hürth (DE); MEISTER, Hartmut, D-50739 Köln (DE)
(74) Vertreter: Bauer, Wulf, Dr.
(86) Internationale Anmeldenummer: DE9800073
(87) Internationale Veröffentlichungsnummer: WO98030175

(56) Entgegenhaltungen:
- EP-A- 0 460 354
- DE-A- 4 407 847
- US-A- 2 191 832
- US-A- 3 712 962
- US-A- 4 169 292
- US-A- 4 957 507

## Beschreibung

Die Erfindung bezieht sich auf eine Mittelohrprothese mit einem rohrförmigen Gehäuse.

Eine derartige Mittelohrprothese ist aus der DE 4 407 847 A1 bekannt. Bei dieser ist das rohrförmige Gehäuse nicht nur an seinem nach außen weisenden Endbereich durch ein Trommelfell geschlossen, sondern es ist auch an dem innenseitigen Endbereich eine Membrane vorgesehen. Diese ist mit einem künstlichen Trommelfell durch ein innenliegendes Übertragungsglied verbunden, das Trommelfell und Membrane nach innen vorspannt. Das Trommelfell hat eine größere Fläche als die Membrane. Die Schallübertragung von der Membrane an die Stapes-Fußplatte erfolgt durch eine kleine Menge körpereigenen Fetts des Patienten. Dieses für die Schallübertragung benötigte Fett liegt außerhalb der Prothese.

Bei dieser vorbekannten Mittelohrprothese ist es einerseits nachteilig, dass der erzielbare Schalldruck an der Stapes-Fußplatte für ein normales Hören nicht ausreichend hoch ist, weil das Flächentransformationsverhältnis verringert wird. Andererseits ist die Schallübertragungsstrecke zwischen Membran und Stapes-Fußplatte auf Dauer mit Unsicherheiten behaftet, weil schon kleinste Lufteinschlüsse im Fett oder ein Schwinden des Fettes und damit kleinste Luftspalte die Schallübertragung stark beeinflussen.

Aus der EP 460 354 B1 ist eine Mittelohrprothese bekannt, die ohne ein eigenes Gehäuse auskommt. Das künstliche Trommelfell ist in einem äußeren Haltering eingespannt, der in eine speziell für ihn vorgesehene, im Felsenbein eingearbeitete Ausnehmung eingesetzt wird. Das Trommelfell hat in seiner Mitte ein Loch, in das ein verdickter Frontbereich eines Ossikelersatzes eingeklipst werden kann. Auf diese Weise kann zunächst der Ossikelersatz im Raum hinter dem künstlichen Trommelfell angeordnet werden. Danach wird das künstliche Trommelfell eingesetzt, das frontseitige Ende des Ossikelersatzes schnappt in die Bohrung des Trommelfells ein.

Weitere Mittelohrprothesen sind aus EP 281 047 B1, DE 2 905 183 C3, DE 2 937 842 C3 und EP 203 785 B1 bekannt.

Aufgabe der Erfindung ist es, die eingangs genannte Mittelohrprothese dahingehend weiterzubilden, dass ein ausreichender Schalldruck im Innenohr vorliegt und eine sichere und dauerhafte Schallübertragung zum Innenohr stets gewährleistet ist.

Ausgehend von der Mittelohrprothese der eingangs genannten Art wird diese Aufgabe gelöst durch die Merkmale des Anspruchs 1.

Die erfindungsgemäße Mittelohrprothese ersetzt die Funktion des schallübertragenden Apparats eines natürlichen Mittelohrs so weit wie möglich vollständig, sie ist dabei nicht auf die Funktion der natürlichen Eustachischen Röhre und/oder der Schleimhaut der Mittelohrräume angewiesen.

Die erfindungsgemäße Mittelohrprothese dient der Versorgung von Patienten mit chronischen Mittelohrentzündungen, die eine normale Schallübertragung dauerhaft schwächen oder behindern, hierzu gehören beispielsweise: Mittelohrschleimhauteiterung, Mittelohrknocheneiterung (= Cholesteatomen-Eiterung), chronische Tubenbelüftungsstörung, Mittelohr-Mißbildungen, dauerhafte Zerstörungen des Schallleitungsapparates durch Traumen und Tumoren etc.. Die Mittelohrprothese ist weder auf eine Schleimhautauskleidung noch auf eine Tubenbelüftung angewiesen. Sie besteht aus einem Gehäuse, das beispielsweise zweiteilig ist und das zumindest keimdicht abschließbar ist. Der Innenraum des Gehäuses ist damit für von außen eindringende Keime nicht erreichbar.

Im Gehäuse ist für die vorläufige Fixierung des Ossikelteils, im folgenden erstes Ossikelteil genannt, die Halteeinrichtung vorgesehen. Diese wird später, vor Abschluß der Operation, entfernt, durchtrennt oder anderweitig außer Funktion gesetzt. Sie kann aber auch, wenn sie elastisch genug ausgebildet ist, bestehen bleiben.

Das erste Ossikelteil ist entweder durch eine künstlich geschaffene Öffnung in der Stapes-Fußplatte hindurchgeführt und regt unmittelbar das Innenohr an, oder es ist auf diese Fußplatte aufgesetzt. Damit ist eine direkte Schallübertragung zum Innenohr gewährleistet. Diese Schallübertragung kann nicht durch Verlust von Ankopplungsfett oder dergleichen beeinträchtigt werden.

Das erste Ossikelteil wird durch ein vorzugsweise künstliches Trommelfell und/oder durch einen elektrischen Antrieb, insbesondere einen elektrodynamischen oder piezoelektrischen Antrieb, bewegt. Ein derartiger elektrischer Antrieb erhält seine Spannungsversorgung durch einen Verstärker, beispielsweise den Verstärker eines Hörgerätes.

Durch das Fenster ist während der Operation der Innenraum des Gehäuses zugänglich. Auf diese Weise können die notwendigen Manipulationen im Gehäuse vorgenommen werden. Insbesondere ist die Halteeinrichtung durch das Fenster zugänglich. Das Fenster wird durch das Abdeckteil keimdicht abgeschlossen.

Für das Fenster sind unterschiedliche Ausbildungen möglich. So kann das Fenster beispielsweise zwischen einem zweiteiligen Gehäuse, das aus einem Übertragungsteil und dem Ankopplungsteil besteht, vorgesehen werden, indem im Überlappungsbereich beider Teile Einschnitte bzw. randseitige Aussparungen vorgesehen sind, die einer gewissen Drehposition der beiden Teile zueinander das Fenster bilden, in einer anderen Drehposition aber vollständig verschließen. Schließlich kommen auch dreiteilige und mehrteilige Gehäuse in Betracht, bei denen das dritte Teil und mögliche weitere Teile dafür bestimmt sind, einen Fensterbereich auszubilden oder zu verschließen, beispielsweise durch einen axial verschiebbaren Zylinder. Hierzu gehören auch ringförmige, zylindrische Teile, die gegenüber dem Ankopplungsteil und/oder Übertragungsteil drehbar sind und wie dieses ein Fenster aufweisen. Durch Drehen wird das Fenster entweder frei oder vollständig verschlossen.

Beim operativen Einsetzen der Mittelohrprothese wird zunächst das Ankopplungsteil eingesetzt, es ist so gut wie möglich mit der Paukenwand des Ohres ringsum das ovale Fenster verbunden. Als besonders bevorzugt hat es sich hier erwiesen, den feien Endbereich des Ankopplungsteils dem Verlauf des jeweiligen individuellen Reliefs der medialen Paukenwand eines Trägers der Mittelohrprothese möglichst genau nachzubilden. Auf diese Weise wird eine gute Passung an die Paukenwand erzielt und es wird dadurch möglich, mit einem geeigneten Verbindungsmaterial einen Abschluß und sicheren Halt des Ankopplungsteils an der Paukenwand zu erzielen.

Wenn das Ankopplungsteil eingesetzt ist, ist im Falle einer Öffnung in der Stapes-Fußplatte entweder diese schon vorgesehen oder wird erst dann ausgeführt. Vorzugsweise wird sie mit einem Laser hergestellt. Sie ist gerade so groß, dass das erste Ossikelteil durch sie hindurchpaßt. Das erste Ossikelteil ist vorzugsweise ein dünner Gold- oder Titandraht. Das erste Ossikelteil wird an der Halteeinrichtung festgelegt oder ist bereits an dieser festgelegt. Diese Festlegung erfolgt so, dass das erste Ossikelteil in einem gewünschten Maß in das Innenrohr hineinragt. Durch die Festlegung ist gewährleistet, dass dieses Maß später nicht unter- oder überschritten wird.

Als besonders vorteilhaft hat es sich erwiesen, dass das Gehäuse der erfindungsgemäßen Mittelohrprothese gegenüber dem umliegenden Gewebe keimdicht, vorzugsweise hermetisch abschließt. Dadurch wird gewährleistet, dass der Innenraum des Gehäuses vor dem Eindringen von Keimen geschützt wird.

Die Lage und Größe des Gehäuses der Mittelohrprothese ist durch umfangreiche Vermessungen von Felsenbeinpräperaten hinlänglich gut bestimmt. Es ergibt sich typischerweise ein mittlerer maximaler Durchmesser von etwa 11 mm und eine maximale Länge von etwa 20 mm.

Vorzugsweise besteht das Gehäuse aus dem Ankopplungsteil und einem Übertragungsteil, die Verbindungsmittel aufweisen, so dass sie miteinander zu dem Gehäuse verbunden werden können. Vorzugsweise ist dabei dem Übertragungsteil ein zweites Ossikelteil zugeordnet. Durch das Fenster kann während des operativen Einsetzens die Verbindung zwischen den beiden Ossikelteilen beobachtet und vorzugsweise auch durchgeführt werden.

Für das Implantieren der Mittelohrprothese hat es sich als günstig erwiesen, am Gehäuse Verbindungsmittel für die mechanische Befestigung des Gehäuses am Felsenbein des Trägers vorzusehen. Zwar ist es möglich und auch durchaus gewollt, den Raum des Mittelohrs um die Prothese herum auszufüllen, so dass die Prothese ansich einen festen Sitz hat. Durch die mechanische Verankerung im Knochen wird während der Operation und auch bei späteren Stößen eine dauerhaft sichere Befestigung gewährleistet.

Bei einer Mittelohrprothese mit einem Trommelfell hat es sich als bevorzugt herausgestellt, das Trommelfell in einem Winkel zur Achse des Gehäuses anzuordnen. Je größer dieser Winkel gewählt wird, um so größer wird die Fläche des Trommelfells. Günstig haben sich Winkel zwischen 40° und 80° zur Achse des Übertragungsteils erwiesen, insbesondere ein Winkel von etwa 53°. Auf diese Weise hat das bevorzugt künstliche Trommelfell eine unrunde Form, insbesondere eine elliptische Form. Dadurch werden Resonanzen vermieden. Insbesondere ist aber auch die große Empfangsfläche für Schall günstig. Es werden typischerweise Flächen von etwa 100 mm² erhalten.

Für das zweite Ossikelteil kann entweder auf handelsübliche Teile zurückgegriffen werden, beispielsweise den von der Firma Richards GmbH unter dem Handelsnamen TILT-TORP-PORP angebotenen Ossikelersatz. Er hat einen Hohlschaft, in den das erste Ossikelteil eingeführt werden kann. Weiterhin hat er an seinem anderen Ende ein Kugelgelenk, wodurch Ausgleichsbewegungen möglich sind. Damit wird eine weitgehend kolbenförmige, in erster Linie eindimensionale Bewegung auf die Innenohrflüssigkeit ermöglicht. Oder es kann ein eigener Prothesenteil entwickelt werden, der diesem mechanischen Prinzip entspricht.

Da die erfindungsgemäße Mittelohrprothese im eingesetzten Zustand einen im wesentlichen hermetisch abgekapselten Innenraum hat, ist eine Belüftung der Mittelohrräume, wie sie im natürlichen Fall durch die Eustachische Röhre realisiert wird, nicht vorhanden. Bei einem rein elektrischen Antrieb kann man auf einen Druckausgleich verzichten. Bei einer Prothese mit einem Trommelfell ist eine künstliche Druckausgleichsvorrichtung aber notwendig. Sie besteht aus einem von außen zugänglichen, beispielsweise hinter dem Ohr verborgenen Außenteil, wie es in ähnlicher Form schon bei knochenverankerten Hörgeräten, die über Körperschall arbeiten, bekannt ist, und einem Innenteil, das beispielsweise im Mastoid untergebracht ist. Dieses Innenteil hat in einer ersten Variante ein Feinfilter, insbesondere ein Filter, das gegen jegliche Art von Mikroben und Viren sperrt, hier kommen insbesondere Hohlfasern als Filtermaterial in Frage, oder es hat eine Druckausgleichsmembran. Hierbei ist eine leicht bewegliche Membran völlig dicht in einem eigenen Gehäuse eingespannt, sie bildet eine absolute Barriere zwischen einer mit der Außenwelt verbundenen Außenkammer und einer Innenkammer, die ihrerseits mit dem Innenraum der Prothese verbunden ist. Außenteil, Innenteil und Mittelohrprothese sind über dünne Schläuche, insbesondere Silikonschläuche, miteinander dicht und dauerhaft verbunden.

Um das individuelle Relief der Paukenwand eines Trägers zu erfassen und demgemäß dem freien Endbereich des Ankopplungsteils den gewünschten Verlauf, also das gewünschte Profil geben zu können, stehen mehrere Möglichkeiten zur Verfügung. Über ein hochauflösendes Spiral-Computertomogramm kann der Verlauf erhalten werden und unmittelbar über eine Zwischenstufe einer Bearbeitungsmaschine für den freien Endbereich zugeleitet werden. Andererseits kann auch über direkte Messungen mit Strahlen das Relief abgetastet und entsprechend die Bearbeitungsmaschine direkt oder indirekt gesteuert werden. Schließlich kann der Verlauf mechanisch abgetastet werden, wozu ein spezielles Abtastgerät entwickelt wurde. Die mit diesem Abtastgerät erhaltenen Daten können zur Steuerung einer Bearbeitungsmaschine eingesetzt werden.

Weitere Vorteile und Merkmale der Erfindung ergeben sich aus den übrigen Patentansprüchen sowie der folgenden Beschreibung von nicht einschränkend zu verstehenden Ausführungsbeispielen. Die Ausführungsbeispiele werden im folgenden anhand der Zeichnung näher erläutert. In dieser zeigen:
- FIG. 1:: eine schnittbildliche Darstellung einer Mittelohrprothese mit angesetzter Druckausgleichvorrichtung,
- FIG. 2:: eine perspektivische Darstellung eines Übertragungsteils mit Blick auf und durch ein Arbeitsfenster,
- FIG. 3:: eine der Darstellung gemäß Figur 2 entsprechende Darstellung eines Ankopplungsteils,
- FIG. 4:: eine Draufsicht auf eine Abdeckplatte,
- FIG. 5:: eine schnittbildliche Darstellung eines Gehäuses in ähnlicher Ansicht wie Figur 1, jedoch nunmehr mit zusätzlich einem elektrodynamischen Antrieb und mit einem über ein Übertragungsglied angeschlossenen externen Verstärker und
- FIG. 6:: eine Darstellung ähnlich Figur 5, jedoch ohne Verstärker und nunmehr ohne Trommelfell, der Antrieb erfolgt ausschließlich piezoelektrisch.

Die Mittelohrprothese hat ein künstliches Trommelfell 20, das im wesentlichen einen ovalen Zuschnitt hat. Der Fläche nach ist es größer als die Fläche eines normalen menschlichen Trommelfells, beispielsweise 1,5 mal so groß. Es ist aus einem für den Einsatz als Prothese biologisch geeigneten Material zugeschnitten, insbesondere aus dem Material Millicell HA der Firma Millipore GmbH, aus GORE-TEX 0,1 mm der Firma W. L. Gore & Assoc. GmbH oder aus nachgiebigem Silikon. Millicell, Millipore, GORE-TEX und Gore sind geschützte Bezeichnungen. Im praktischen Einsatz ist das Trommelfell 20 epithelialisiert.

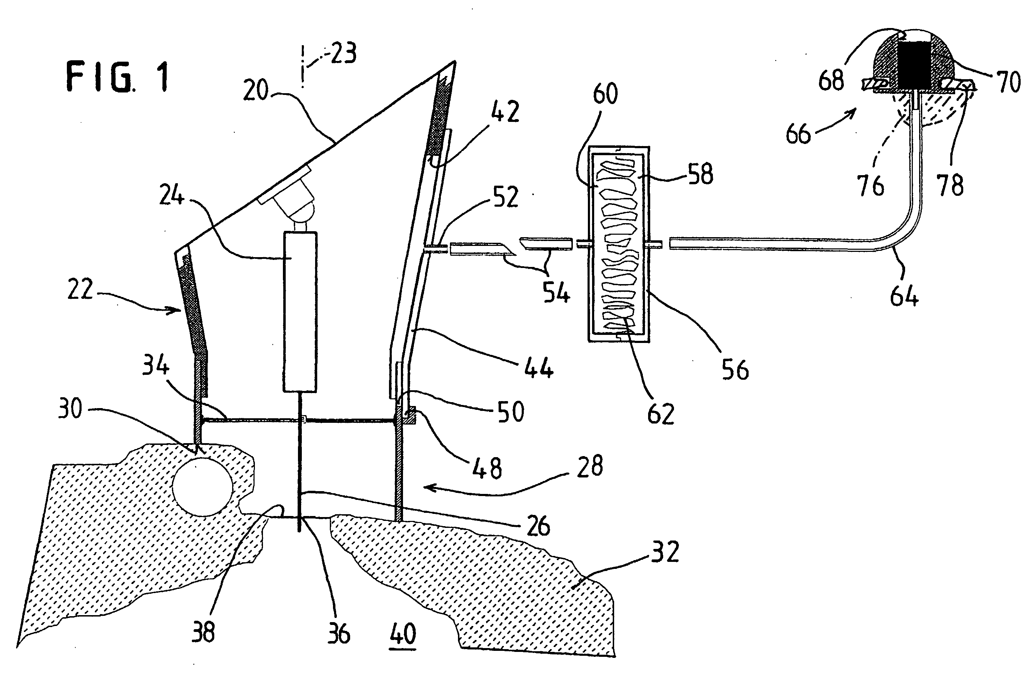

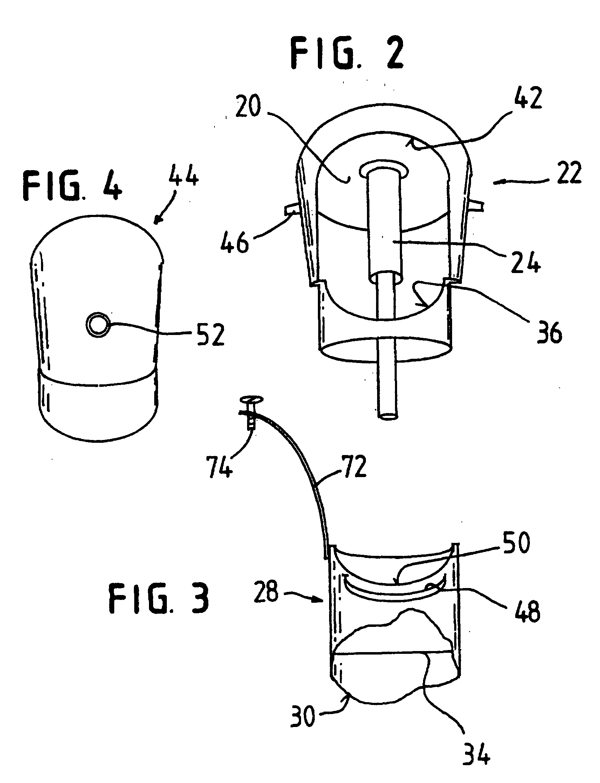

Das Trommelfell 20 ist dicht verbunden mit einem im gezeigten Ausführungsbeispiel nach den Figuren zweiteilig ausgebildeten Gehäuse, konkret ist es mit einem Übertragungsteil 22 verbunden. Dieses ist im wesentlichen rohrförmig. Es hat einen im wesentlichen zylindrischen Bereich, der in Figur 2 unten dargestellt ist und einen sich von diesem aus erweiternden Bereich, der im wesentlichen auf einem Kegelmantel verläuft. Der Durchmesser des zylindrischen Bereichs liegt bei etwa 8 mm, der sich erweiternde Bereich hat entsprechende Abmessungen von etwa 11 mm. Wie insbesondere Figur 1 zeigt, befindet sich das Trommelfell 20 in einer Ebene, sie verläuft in einem Winkel 35° zu einer Längsachse 23 des Übertragungsteils 22. Durch die Schrägstellung des Trommelfells 20 hat das Trommelfell 20 eine gegenüber dem normalen Ohr größere Fläche.

Auf der Innenseite des Trommelfells 20, vorzugsweise im Flächenmittelpunkt, ist ein zweites Ossikelteil 24 dauerhaft befestigt. Im konkreten Ausführungsbeispiel wird ein handelsüblicher Ossikelersatz der Firma Richards GmbH eingesetzt. Er hat in Nähe des Trommelfells 20 ein Kugelgelenk, darunter befindet sich ein Hohlschaft für die Aufnahme eines noch zu besprechenden ersten Ossikelteils. Das zweite Ossikelteil 24 verläuft unterhalb des Kugelgelenkes zentrisch zur Mittelachse des Übertragungsteils 22.

Weiterhin gehört zum Gehäuse ein Ankopplungsteil 28. Es ist insgesamt etwas kleiner als das Übertragungsteil 22 und mit diesem gemeinsam so ausgebildet, das beide Teile einfach, dicht und dauerhaft miteinander verbunden werden können, insbesondere durch Stecken in Längsrichtung und Kleben. Das Ankopplungsteil 28 ist im wesentlichen zylindrisch, sein Durchmesser liegt ebenfalls bei etwa 6 mm. Es hat einen freien Endbereich 30, der einen Verlauf hat, welcher dem jeweiligen individuellen Relief der Paukenwand eines Träger der Mittelohrprothese möglichst genau nachgebildet ist. Der sich dadurch ergebende, individuelle Verlauf ist insbesondere aus Figur 3 ersichtlich. Wie bereits oben ausgeführt wurde, gibt es mehrere Verfahren, um den Verlauf des individuellen Reliefs der Paukenwand zu ermitteln bzw. abzutasten. Hierzu wird auch auf die Patentanmeldung "Vorrichtung zum mechanischen Abtasten und Erfassen des Verlaufs der Paukenwand eines Mittelohrs" des selben Anmelders und vom selben Anmeldetag verwiesen, der Offenbarungsgehalt dieser Anmeldung wird zum Gegenstand der Offenbarung der vorliegenden Anmeldung gemacht.

Der freie Endbereich 30 ist demgemäß so ausgeführt, dass er präzise an der Paukenwand 32 anliegt, wie dies aus Figur 1 ersichtlich ist. Auf diese Weise ist ein hermetisch dichter Abschluß an dieser Stelle möglich, es kann dünnflüssiges Befestigungs- und Dichtmaterial verwendet werden, um die Verbindung zwischen dem freien Endbereich 30 und der Paukenwand 32 zu realisieren.

Im Ankopplungsteil 28 ist eine Halteeinrichtung 34 für das erste Ossikelteil 26 vorgesehen. Letzteres ist als ein dünner Golddraht ausgeführt, der durch eine sehr kleine, künstlich geschaffene Öffnung 36 in einer Stapes-Fußplatte 38 gerade passend hindurch reicht und somit mit seinem unteren, freien Endbereich in das Innenohr 40 hineinragt. Im gezeigten Ausführungsbeispiel ist die Halteeinrichtung 34 ein im wesentlichen diagonal verlaufender Draht, an dem das erste Ossikelteil 26 festliegt bzw. festlegbar ist. Während der Implantation der Mittelohrprothese wird die Halteeinrichtung 34 durchtrennt, so dass sich das mit dem zweiten Ossikelteil 24 verbundene erste Ossikelteil 26 durch sie frei hin- und her bewegen kann.

Bei dem hier gezeigten Ausführungsbeispiel der Mittelohrprothese ist für die Verbindung der beiden Ossikelteile 24, 26 und auch für das Durchtrennen der Halteeinrichtung 34 ein Zugang in den Innenraum notwendig. Andere Konstruktionen ohne einen derartigen Zugang sind möglich. Für diesen Zugang ist im Übertragungsteil 22 ein Fenster 42 vorgesehen, das durch eine Abdeckplatte 44 verschließbar ist. Sie ist in Figur 4 dargestellt. Es sind geeignete mechanische Haltemittel vorgesehen, um die Abdeckplatte 44 präzise und auch dicht am Übertragungsteil 22 und am Ankopplungsteil 28 festlegen zu können. So sind im gezeigten Ausführungsbeispiel Dorne 46 zur Fixierung der Abdeckplatte 44 vorgesehen, am Ankopplungsteil 28 wird durch einen Vorsprung eine Nut 48 für die Aufnahme der Abdeckplatte 44 gebildet. Wie Figur 3 zeigt, hat vorzugsweise auch das Ankopplungsteil 28 in seinem oberen Bereich eine Aussparung 50, die auch als untere Fensteraussparung bezeichnet wird.

Wie Figur 4 zeigt, hat die Abdeckplatte 44 einen Anschluß 52. Er ist für einen Schlauch 54 vorgesehen, beides ist auch aus Figur 1 ersichtlich. Der Schlauch 54 führt zu einem Innenteil 56 einer Druckausgleichsvorrichtung. Dieses Innenteil 56 ist als ein dosenförmiges Gehäuse ausgeführt, es hat zwei Kammern, nämlich eine Außenkammer 58 und eine Innenkammer 60. Beide sind durch eine sehr flexible Membran 62 hermetisch voneinander getrennt. In einer anderen Ausführung sind sie durch ein Feinfilter, das auch gegen Bakterien und Mikroben dicht ist, aber luftdurchlässig ist, voneinander separiert. Als Feinfilter kommen hier insbesondere Hohlfaserfilter in Frage.

Über einen weiteren Schlauch 64 ist die Außenkammer 58 mit einem Außenteil 66 verbunden. Es ist in einem Knochen 76 verankert und teilweise von außen zugänglich, siehe Haut 78. Es wird hier eine Konstruktion verwandt, wie sie ähnlich ist bei sogenannten knochenleitenden Hörgeräten. Das Außenteil 66 hat eine Ausnehmung 68, in die ein Filter 70 eingesetzt ist. Dieses ist vorzugsweise austauschbar. Das Filter 70 verhindert, dass Wasser, grober Schmutz usw. in den Schlauch 64 gelangen kann. Die Barriere gegen Keime und dergleichen wird durch die Membran 62 bzw. das an ihre Stelle tretende Feinfilter erreicht. Auf die Anmeldung desselben Anmelders mit dem gleichen Anmeldetag "Druckausgleichsvorrichtung als prothetischer Ersatz für eine Eustachische Röhre" wird Bezug genommen, der Offenbarungsgehalt dieser Anmeldung gehört ebenfalls zum Offenbarungsgehalt der vorliegenden Anmeldung.

Als Material für die Teile 22, 26, 28 und 44 sowie 56, 66 kommt insbesondere Titan in Betracht. Gemäß Figur 3 ist am Ankopplungsteil eine Lasche 72 befestigt, über die eine Befestigung am Felsenbein erfolgt, hierzu ist eine Schraube 74 vorgesehen.

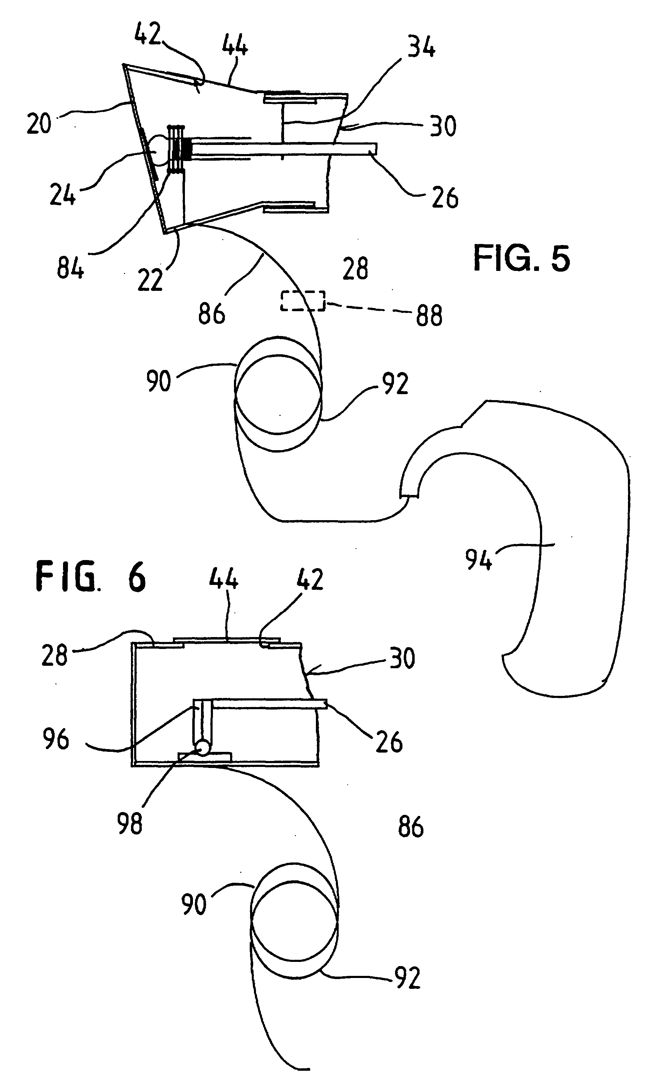

Im zweiten Ausführungsbeispiel, das in Figur 5 dargestellt ist, wird ein Gehäuse mit einem künstlichen Trommelfell 20 eingesetzt, das dem Gehäuse des ersten Ausführungsbeispiels entspricht. Geändert ist gegenüber der ersten Ausführung, dass in der rohrförmigen Führung des zweiten Ossikelteils 24 ein ferromagnetischer Kern 82, insbesondere ein Kern aus einem Ferritmaterial, angeordnet ist. Um den Bereich des zweiten Ossikelteils 24 außerhalb des Kerns 82 ist eine mit dem Gehäuse verbundene Spule 84 aufgebracht. Das zweite Ossikelteil 24 ist aus einem Kunststoff oder einem anderen, nicht leitenden Material hergestellt. Spule 84 und Kern 82 bilden zusammen einen elektrodynamischen Antrieb. Die Spule 84 ist über eine Zuleitung 86 und gegebenenfalls unter Zwischenschaltung eines implantierten Verstärkers 88 mit einer Sekundärspule 90 eines Transformators verbunden. Die Sekundärspule 90 befindet sich unterhalb der Haut 78, während die zugehörige Primärspule 92 sich außerhalb der Haut 78 befindet. Die Primärspule 92 ist mit dem Ausgang eines Hörgerätes 94 verbunden.

Beim praktischen Betrieb empfängt das im Hörgerät 94 eingebaute oder diesem zugeordnete Mikrophon Schallinformationen. Sie werden im Hörgerät 94 verstärkt und gegebenenfalls aufbereitet. Die verstärkten Signale werden der Primärspule 92 zugeleitet. Von dort werden sie auf die Sekundärspule 90 und damit auf die Spule 84 übertragen.

Um die Leistungsübertragung innerhalb des Transformators 90, 92 zu verbessern, empfiehlt es sich, im Hörgerät 94 die Schallinformation auf eine hohe Frequenz zum Beispiel von ein Megahertz umzusetzen. Dies geschieht ähnlich der Trägerfrequenztechnik bei Telefonsystemen oder in entsprechender Weise.

Nach wie vor wird wie im ersten Ausführungsbeispiel die Bewegung des Trommelfells 20 auf die beiden Ossikelteile 24, 26 übertragen. Es liegen also zwei Bewegungsantriebe vor. Einerseits werden die Ossikelteile 24, 26 durch das Trommelfell 20 und damit durch den unmittelbar das Trommelfell 20 erreichenden Schall bewegt, andererseits durch den oben beschriebenen elektrodynamischen Antrieb. Der elektrodynamische Antrieb kann dabei nur für gewisse Frequenzbereiche, beispielsweise nur den hohen Frequenzbereich, die Bewegung unterstützen. Über den elektrodynamischen Antrieb wird dann bevorzugt eine Schwerhörigkeit, die nur in gewissen Frequenzbereichen vorliegt, ausgeglichen. Der elektrodynamische Antrieb kann aber auch im gesamten Frequenzbereich aktiv sein.

Im Ausführungsbeispiel nach Figur 6 sind mehrere entscheidende Änderungen vorgenommen. Das Gehäuse ist nunmehr nur noch einteilig, es besteht nur noch aus dem Ankopplungsteil 28, in dem das Fenster 42 vorgesehen ist, das durch die Abdeckplatte 44 abgedeckt ist. Anstelle eines Trommelfells 20 ist das Gehäuse fest an dem Endbereich verschlossen, der dem freien Endbereich 30 gegenüberliegt. Das Gehäuse ist also im wesentlichen becherförmig. Der Verschluß am Endbereich kann als Fenster anstelle des gezeichneten Fensters 42 dienen.

Es ist auch nur ein erstes Ossikelteil 26 vorgesehen, ein zweites Ossikelteil entfällt. Das erste Ossikelteil ist über ein quer zu ihm verlaufendes piezoelektrisches Element 96 mit der Innenwand des Ankopplungsteils 28 verbunden. Hier ist eine Halterung 98 vorgesehen. Das piezoelektrische Element 96 hat die Form einer länglichen Platte, die hier als Doppelplatte ausgeführt ist. Bei entsprechender elektrischer Anregung führt es Bewegungen in Längsrichtung des ersten Ossikelteils 26 durch. Es ist über die Zuleitung 86 an die Sekundärspule 90 eines Transformators angeschlossen. Die Übertragung und Verbindung mit einem Hörgerät 94 geschieht wie im Ausführungsbeispiel nach Figur 5.

Im Gegensatz zu dem Ausführungsbeispiel nach Figur 5 übernimmt der elektrische Antrieb nunmehr die komplette Schallanregung des Innenohrs, da kein Trommelfell mehr vorgesehen ist. Selektive Hörprobleme des Trägers der Ohrprothese können dadurch ausgeglichen werden, dass im Hörgerät 94 entsprechende Vorkehrungen getroffen sind.

## Patentansprüche

1. Mittelohrprothese mit einem rohrförmigen Gehäuse, das ein Ankopplungsteil (28) aufweist, **dadurch gekennzeichnet, dass**
- das Ankopplungsteil (28) einen freien Endbereich (30) aufweist, der ausgebildet ist für eine Anlage an der medialen Paukenwand (32) eines Ohrs,
- das Gehäuse eine in seinem Innenraum befindliche Halteeinrichtung (34) für ein durch eine kleine, künstlich geschaffene Öffnung (36) in einer Stapes-Fußplatte (38) ragendes oder auf dieser Fußplatte aufsetzendes Ossikelteil (26) aufweist,
- das Gehäuse mit einem Fenster (42) versehen ist, durch das die Halteeinrichtung (34) zugänglich ist und
- ein Abdeckteil (44) für das Fenster (42) vorgesehen ist

2. Mittelohrprothese nach Anspruch 1, **dadurch gekennzeichnet, dass** der freie Endbereich des Ankopplungsteils (28) einen Verlauf hat, der dem jeweiligen individuellen Relief eines Trägers der Mittelohrprothese möglichst genau nachgebildet ist.

3. Mittelohrprothese nach Anspruch 1, **dadurch gekennzeichnet, dass** ein vorzugsweise künstliches Trommelfell (20) vorgesehen ist, das mit dem Gehäuse verbunden ist, dass ein zweites Ossikelteil (24) vorgesehen ist, das mit der Innenfläche des Trommelfells (20) verbunden ist und dass das erste Ossikelteil (26) und das zweite Ossikelteil (24) Mittel für ihre Verbindung aufweisen.

4. Mittelohrprothese nach Anspruch 3, **dadurch gekennzeichnet, dass** das Trommelfell (20) in einem Winkel von 85° bis 40°, vorzugsweise 53°, zur Achse des Übertragungsteil (22) verläuft, insbesondere, dass das Trommelfell (20) einen elliptischen Zuschnitt hat.

5. Mittelohrprothese nach Anspruch 1, **dadurch gekennzeichnet, dass** ein elektrischer Antrieb, insbesondere ein elektrodynamischer oder piezoelektrischer Antrieb vorgesehen ist, der im Gehäuse angeordnet ist und mit dem ersten Ossikelteil (26) bewegungsverbunden ist.

6. Mittelohrprothese nach Anspruch 1, **dadurch gekennzeichnet, dass** am Gehäuse Verbindungsmittel (72, 74) für die mechanische Befestigung des Gehäuses an einem Knochen, insbesondere am Felsenbein des Trägers der Mittelohrprothese, vorgesehen sind.

7. Mittelohrprothese nach Anspruch 1, **dadurch gekennzeichnet, dass** das erste Ossikelteil (26) mit der Halteeinrichtung (34) lösbar verbunden ist.

8. Mittelohrprothese nach Anspruch 1, **dadurch gekennzeichnet, dass** bei dichtem Abschluß am freien Endbereich (30) des Ankopplungsteils (28), insbesondere beim Einsatz an der Paukenwand (32) eines Ohrs, der Innenraum des Gehäuses keimdicht, vorzugsweise luftdicht abgeschlossen ist.

9. Mittelohrprothese nach Anspruch 1, **dadurch gekennzeichnet, dass** der Innenraum des Gehäuses über einen Schlauch (54) mit einer Druckausgleichsvorrichtung verbunden ist.

10. Mittelohrprothese nach Anspruch 1, **dadurch gekennzeichnet, dass** das Gehäuse aus dem Ankopplungsteil (28) und einem Übertragungsteil (22) zusammengesetzt ist.

## Claims

1. Middle ear prosthesis with a tubular housing which housing is provided with a coupling part (28) **characterized in that**
- the coupling part (28) has a free end area (30) designed for mounting on the medial eardrum wall (32) of an ear,
- the housing has in its inner space a holding device (34) for an ossicle part (26) that is projecting through a small, artificial opening (36) in a stapes base plate (38) or that is sitting on said base plate,
- the housing is provided with a window (42) through which the holding device (34) may be reached and
- a cover part (44) for the window (42).

2. Middle ear prosthesis according to claim 1, **characterized in that** the free end area of the coupling part (28) has a course imitating as accurately as possible the relief of the medial eardrum wall of the patient wearing the middle ear prosthesis.

3. Middle ear prosthesis according to claim 1, **characterized in that** a preferably artificial eardrum (20) connected to the housing is provided, that a second ossicle part (24) connected to the inner surface of the eardrum (20) is provided and that the first ossicle part (26) and the second ossicle part (24) have means for their interconnection.

4. Middle ear prosthesis according to claim 3, **characterized in that** the eardrum (20) is running in an angle of 85° to 40°, preferably 53°, to the axis of the transmission part (22), particularly that the eardrum (20) has an elliptic blank.

5. Middle ear prosthesis according to claim 1, **characterized in that** an electric actuation, particularly an electrodynamic or piezoelectric actuation is provided, said actuation being arranged in the housing and being connected in movement with the first ossicle part (26).

6. Middle ear prosthesis according to claim 1, **characterized in that** the housing is provided with connecting means (72, 74) for the mechanical fixation of the housing on a bone, particularly on the petrous bone of the wearer of the middle ear prosthesis.

7. Middle ear prosthesis according to claim 1, **characterized in that** the first ossicle part (26) is detachably connected to the holding device (34).

8. Middle ear prosthesis according to claim 1, **characterized in that**, when the free end area (30) of the coupling part (28) is tightly closed, particularly when it is mounted on the eardrum wall (32) of an ear, the inner space of the housing is sealed against germs and preferably against air.

9. Middle ear prosthesis according to claim 1, **characterized in that** the inner space of the housing is connected via a tube (54) to a pressure compensating device.

10. Middle ear prosthesis according to claim 1, **characterized in that** the housing is composed of the coupling part (28) and of the transmission part (22).

## Revendications

1. Prothèse de l'oreille moyenne avec un boîtier tubulaire qui présente une partie d'accouplement (28), **caractérisée par le fait que**
- la partie d'accouplement (28) présente une zone terminale libre (30) qui est réalisée pour un appui sur la paroi tympanique médiale (32) d'une oreille,
- le boîtier présente un dispositif de retenu (34) situé dans son intérieur et destiné à une partie d'osselet (26) qui s'étend à travers une petite ouverture (36) créée de manière artificielle dans une plaque de base d'étrier (38) ou qui est assise sur cette plaque de base,
- le boîtier est pourvu d'une fenêtre (42) à travers laquelle le dispositif de retenu (34) est accessible, et
- qu'une partie de recouvrement (44) est prévue pour la fenêtre (42).

2. Prothèse de l'oreille moyenne selon la revendication 1, **caractérisée par le fait que** la zone terminale libre de la partie d'accouplement (28) présente une allure qui est reproduite d'une façon aussi précise que possible selon le relief individuel respectif de la paroi tympanique médiale d'une personne qui porte la prothèse de l'oreille moyenne.

3. Prothèse de l'oreille moyenne selon la revendication 1, **caractérisée par le fait qu'**un tympan (20) de préférence artificiel est prévu qui est relié au boîtier, qu'une deuxième partie d'osselet (24) est prévue qui est reliée à la face intérieure du tympan (20), et que la première partie d'osselet (26) et la deuxième partie d'osselet (24) présentent des moyens pour leur jonction.

4. Prothèse de l'oreille moyenne selon la revendication 3, **caractérisée par le fait que** le tympan (20) s'étend à un angle compris entre 85° et 40°, de préférence de 53°, par rapport à l'axe de la partie de transmission (22), en particulier que le tympan (20) présente une coupe elliptique.

5. Prothèse de l'oreille moyenne selon la revendication 1, **caractérisée par le fait que** l'on prévoit un entraînement électrique, en particulier un entraînement électrodynamique ou piézoélectrique qui est disposé dans le boîtier et relié à mouvement à la première partie d'osselet (26).

6. Prothèse de l'oreille moyenne selon la revendication 1, **caractérisée par le fait que** l'on prévoit sur le boîtier des moyens de jonction (72, 74) pour la fixation mécanique du boîtier sur un os, en particulier sur le rocher de la personne portant la prothèse de l'oreille moyenne.

7. Prothèse de l'oreille moyenne selon la revendication 1, **caractérisée par le fait que** la première partie d'osselet (26) est reliée de manière amovible au dispositif de retenu (34).

8. Prothèse de l'oreille moyenne selon la revendication 1, **caractérisée par le fait que** dans le cas d'une fermeture étanche sur la zone terminale libre (30) de la partie d'accouplement (28), en particulier lors de l'application sur la paroi tympanique (32) d'une oreille, l'espace intérieur du boîtier est fermé d'une manière étanche aux germes, de préférence étanche à l'air.

9. Prothèse de l'oreille moyenne selon la revendication 1, **caractérisée par le fait que** l'espace intérieur du boîtier est relié par un tuyau (54) à un dispositif de compensation de pression.

10. Prothèse de l'oreille moyenne selon la revendication 1, **caractérisée par le fait que** le boîtier est composé de la partie d'accouplement (28) et d'une partie de transmission (22).
